# EUROPEAN PATENT SPECIFICATION

(11) **EP 2 642 980 B1**
(45) Date of publication and mention of the grant of the patent: **08.04.2020**
(21) Application number: 11794307.6
(22) Date of filing: 23.11.2011
(51) Int. Cl.: A61K 31/137, A61K 9/14, A61K 9/00, A61P 25/18

(54) **PEDIATRIC FORMULATION**
PÄDIATRISCHE FORMULIERUNG
FORMULATION PÉDIATRIQUE

(30) Priority: 23.11.2010 US 416662 P
(43) Date of publication of application: 02.10.2013
(73) Proprietor: Amgen Inc., South San Francisco, CA 94080 (US)
(72) Inventor: BI, Mingda, Simi Valley, California 93063 (US); ALVAREZ-NUNEZ, Fernando, Newbury Park, California 91320 (US); ALVAREZ, Francisco Javier, Newbury Park, California 91320 (US)
(74) Representative: Grünecker Patent- und Rechtsanwälte PartG mbB
(86) International application number: PCT/US2011/062085
(87) International publication number: WO 2012/071535

(56) References cited:
- WO-A1-2005/034928
- WO-A2-2008/027522
- WO-A2-2008/064202

## Description

### FIELD OF THE INVENTION

The present invention is directed to pediatric formulation of (*R*)-*N-*[-1-(1-naphthyl)-ethyl]-3-[3-(trifluoromethyl)phenyl]propan-1-amine hydrochloride (hereinafter referred to as Cinacalcet HCl) and method of administering the same.

### BACKGROUND OF THE INVENTION

Cinacalcet HCl is an allosteric modulator of calcium-sensing receptor and is approved for the treatment of secondary hyperparathyroidism (sHPT) in ESRD (end-stage renal disease) patients. Its main function is to lower PTH and calcium in both ESRD and chronic kidney disease (CKD) patients. Currently, Cinacalcet HCl is marketed only in tablet form. Although tablet is one of the most common drug delivery platforms, some patients, such as children, with specific disorders, may have compliance issues in taking this dosage form. An oral disintegrating tablet (ODT) dosage form was created for these patients a decade ago. This dosage form greatly enhanced the patient compliance. However, ODT technologies require specific manufacturing and packaging equipment, which is expensive. Furthermore, most ODT technologies have low production rate which further increases the manufacturing cost. In addition, ODT is soft, hygroscopic and difficult to handle. Additionally, an ODT dosage form may not work for all drug substances especially for those that show unfavorable organoleptic properties, such as bitter taste, strong odor, and numbness. WO2008/064202 discloses pharmaceutical compositions comprising Cinacalcet-HCl and colloidal silicone dioxide. WO2005/034928 discloses pharmaceutical compositions comprising Cinacalcet-HCl and about 0.05% to about 5% by weight, relative to the total weight of the composition, of at least one additive chosen from colloidal silicon dioxide, magnesium stearate, talc and the like, and mixtures of any of the foregoing.

Therefore, novel formulations and delivery platform of Cinacalcet HCl are needed to enhance the pediatric patient compliance. The present invention fulfils this and related needs.

### SUMMARY OF THE INVENTION

The present invention provides a hard shell capsule containing a granular powder formulation of Cinacalcet HCl. This powder formulation can be sprinkled on and mixed with food or drinks and then administered orally to the pediatric patients. This mode of administration overcomes compliance issues in pediatric patients that are associated with administering tablet and capsule formulations. Initial studies (see Table 2) found that Cinacalcet HCl could not be completely sprinkled out of the capsules. Sometimes more than 50% Cinacalcet HCl was retained in the capsule thereby resulting in severely under-dosing of the patients. Applicants have surprisingly found that an anti-adherent agent or glidant, such as silicon dioxide, when admixed in appropriate proportions with Cinacalcet HCl containing powder formulation, can dramatically reduce the drug retention in the capsules after sprinkling, thereby making the present mode of administering Cinacalcet HCl, in therapeutic amount, to pediatric patients possible.

Accordingly, in one aspect, this invention is directed to a powder formulation, the powder comprising a therapeutically effective amount of (*R*)-*N-*[-1-(1-naphthyl)ethyl]-3-[3-(trifluoromethyl)phenyl]propan-1-amine hydrochloride admixed with at least about 2% to about 5% w/w of silicon dioxide. Preferably, the amount of silicon dioxide is between at least about 3% to about 5% w/w. More preferably, the amount of silicon dioxide is between at least about 4% to about 5% w/w. Most preferably, the amount of silicon dioxide is about 5% w/w by weight in the formulation. Preferably, the powder formulation is in granulated form.

In a second aspect, this invention is directed to a powder formulation, the powder consisting essentially of a therapeutically effective amount of (R)-N-[-1-(1-naphthyl)-ethyl]-3-[3-(trifluoromethyl)phenyl]propan-1-amine hydrochloride admixed with at least about 0.5% to about 5%, preferably 2% to about 5 % w/w of silicon dioxide. More preferably, the amount of silicon dioxide is between at least about 3% to about 5 % w/w. Most preferably, the amount of silicon dioxide is about 5% w/w by weight in the formulation. Preferably, the powder formulation is in granulated form.

In a third aspect, this invention is directed to a hard shell capsule containing a powder, the powder comprising a therapeutically effective amount of (R)-N-[-1-(1-naphthyl)ethyl]-3-[3-(trifluoromethyl)phenyl]propan-1-amine hydrochloride admixed with at least about 2% to about 5% w/w of silicon dioxide. Preferably, the amount of silicon dioxide is between at least about 3% to about 5% w/w. More preferably, the amount of silicon dioxide is between at least about 4% to about 5% w/w. Most preferably, the amount of silicon dioxide is about 5% w/w by weight in the formulation. Preferably, the powder formulation is in granulated form.

In a fourth aspect, this invention is directed to a hard shell capsule containing a powder, the powder consisting essentially of a therapeutically effective amount of (*R*)-*N-*[-1-(1-naphthyl)ethyl]-3-[3-(trifluoromethyl)phenyl]propan-1-amine hydrochloride admixed with at least about 0.5% to about 2%, preferably 2% to about 5% w/w of silicon dioxide. Preferably, the amount of silicon dioxide is between at least about 4% to about 5% w/w. More preferably, the amount of silicon dioxide is between at least about 4.5% to about 5% w/w. Most preferably, the amount of silicon dioxide is about 5% w/w by weight in the formulation. Preferably, the powder formulation is in granulated form.

In a fifth aspect, this invention is directed to use in a method of treating secondary hyperparathyroidism in a pediatric patient comprising administering to the patient any of the above disclosed powder formulations. Preferably, the powder formulation is mixed with food or drinks and administered to the patient.

### DETAILED DESCRIPTION

In the context of the foregoing and subsequent description, including the claims, the term "granulated" will be understood to refer to powder which has a particle size within the range of about 1 µm to about 2000 µm in diameter. Preferably, the particle size distribution in the powder formulation is as follows: the range of about 0.1% w/w to about 5% w/w has a particle size above 600 µm, about 0.5% w/w to about 5% w/w has a particle size above 425 µm, about 1% w/w to about 10% w/w has a particle size above 250 µm, about 5% w/w to about 30% w/w has a particle size above 180 µm, about 5% w/w to about 20% w/w has a particle size above 150 µm, about 8% w/w to about 35% w/w has a particle size above 106 µm, about 10% w/w to about 40% w/w has a particle size above 75 µm and about 10% w/w to about 50% w/w has a particle size below 75 µm in diameter. More preferably, the particle size distribution in the powder formulation is as follows: about 0.54% w/w has a particle size above 600 µm, about 1.01% w/w has a particle size above 425 µm, about 4.80% w/w has a particle size above 250 µm, about 10.5% w/w has a particle size above 180 µm, about 9.9% w/w has a particle size above 150 µm, about 18.4% w/w has a particle size above 106 µm, about 23.1% w/w has a particle size above 75 µm, and about 31.8% w/w has a particle size below 75 µm.

For the avoidance of any doubt when using the term % weight per weight (% w/w) of formulation for the constituents of the formulation, those in the art understand that within a unit weight of the formulation, a certain percentage of the constituent by weight will be present, for example a 1% w/w formulation will contain within a 100 g weight of formulation, 1 g of the constituent. By way of further illustration:

| % w/w of component X in a formulation | Weight of X in 1 g of formulation |
|---|---|
| 30% | 300 mg |
| 20% | 200 mg |
| 10% | 100 mg |
| 5% | 50 mg |
| 1% | 10 mg |

The powder formulation of the present invention may further comprise filler(s), binder(s), disintegrant(s), flavorant(s), sweetener(s), and other suitable excipients well known in the formulation art.

Suitable fillers, include but are not limited, to starches, lactose, mannitol, Pearlitol(TM) SD 200, cellulose derivatives, sugar and the like. Different grades of lactose include, but are not limited, to lactose monohydrate, lactose DT (direct tableting), lactose anhydrous, Flowlac(TM) (available from Meggle products), Pharmatose(TM) (available from DMV) and others. Different grades of starches include, but are not limited to, maize starch, potato starch, rice starch, wheat starch, pregelatinized starch (commercially available as PCS PC10 from Signet Chemical Corporation) and Starch 1500, Starch 1500 LM grade (low moisture content grade) from Colorcon, fully pregelatinized starch (commercially available as National 78-1551 from Essex Grain Products) and others. Different cellulose compounds that can be used include crystalline cellulose and powdered cellulose. Examples of crystalline cellulose products include but are not limited to CEOLUS(TM) KG801, Avicel(TM) PH 101, PH102, PH301, PH302 and PH-F20, microcrystalline cellulose 114, and microcrystalline cellulose 112. Other useful fillers include, but are not limited to, carmellose, sugar alcohols such as mannitol, sorbitol and xylitol, calcium carbonate, magnesium carbonate, dibasic calcium phosphate, and tribasic calcium phosphate. Preferably, the filler is pregelatinized Starch 1500 and microcrystalline cellulose, Avicel pH 102. Preferably, the amount of Starch 1500 and Avicel pH 102 is between at least about 1.0% to about 99.0% w/w. More preferably, the amount of filler is between at least about 5.0% to about 80% w/w. Most preferably, the amount of Starch 1500 and Avicel pH 102 is about 10.29% and 51.39% w/w by weight, respectively in the formulation.

Suitable binders include, but are not limited to, hydroxypropylcellulose (Klucel(TM)-LF), hydroxypropyl methylcellulose or hypromellose (Methocel(TM)), polyvinylpyrrolidone or povidone (PVP-K25, PVP-K29, PVP-K30, PVP-K90), plasdone S 630 (copovidone), powdered acacia, gelatin, guar gum, carbomer (e.g. carbopol), methylcellulose, polymethacrylates, and starch. Preferably, the binder is Povidone K29/32. Preferably, the amount of binder is between at least about 1.0 % to about 10.0 % w/w. More preferably, the amount of binder is between at least about 2.0% to about 6.0% w/w. Most preferably, the amount of binder is about 3.14% w/w by weight in the formulation.

Suitable disintegrants include, but are not limited to, carmellose calcium (Gotoku Yakuhin Co., Ltd.), carboxy methylstarch sodium (Matsutani Kagaku Co., Ltd., Kimura Sangyo Co., Ltd., etc.), croscarmellose sodium (FMC-Asahi Chemical Industry Co., Ltd.), crospovidone, examples of commercially available crospovidone products including but not limited to crosslinked povidone, Kollidon(TM) CL [manufactured by BASF (Germany)], Polyplasdone(TM) XL, XI-10, and INF-10 [manufactured by ISP Inc. (USA)], and low-substituted hydroxypropylcellulose. Examples of low-substituted hydroxypropylcelluloses include but are not limited to low-substituted hydroxypropylcellulose LH11, LH21, LH31, LH22, LH32, LH20, LH30, LH32 and LH33 (all manufactured by Shin-Etsu Chemical Co., Ltd.). Other useful disintegrants include sodium starch glycolate and starch. Preferably, the disintegrant is polyplasdone XL. Preferably, the amount of disintegrant is between at least about 1.0% to about 8.0% w/w. More preferably, the amount of disintegrant is between at least about 1.5% to about 5.0% w/w. Most preferably, the amount of disintegrant is about 1.89% w/w by weight in the formulation.

Suitable anti-adherent agents or glidants, include but are not limited to, talc, silica derivatives, colloidal silicon dioxide, Syloid 244 FP and the like, and mixtures thereof. Preferably, the anti-adherent agent is Syloid 244 FP. Preferably, the amount of anti-adherent is between at least about 0.5% to about 5.0% w/w. More preferably, the amount of anti-adherent is between at least about 3.0% to about 5.0% w/w. Most preferably, the amount of anti-adherent is about 5.0% w/w by weight in the formulation.

Suitable lubricants that can be used include, but are not limited to, stearic acid and stearic acid derivatives such as magnesium stearate, calcium stearate, zinc stearate, sucrose esters of fatty acid, polyethylene glycol, talc, sodium stearyl fumarate, zinc stearate, castor oils, and waxes. Preferably, the lubricant is magnesium stearate. Preferably, the amount of lubricant is between at least about 0.10% to about 2.0% w/w. More preferably, the amount of lubricant is between at least about 0.25% to about 1.0% w/w. Most preferably, the amount of lubricant is about 0.5% w/w by weight in the formulation.

Suitable souring agents include, but are not limited to, citric acid, tartaric acid, malic acid and the like.

Suitable sweeteners include, but are not limited to, artificial sweeteners such as saccharin sodium, sucralose, acesulfame K, glycyrrhizin dipotassium, aspartame, stevia, thaumatin and the like.

Suitable coloring agents include, but are not limited to, food dyes such as Food Yellow No. 5, Food Red No. 2, Food Blue No. 2 and the like, as well as food lake dyes, red iron oxide and the like.

Flavors incorporated in the composition may be chosen from synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leaf oil, oil of nutmeg, oil of sage, oil of bitter almonds and cassia oil. Also useful as flavors are vanilla, citrus oil, including lemon, orange, grape, lime and grapefruit, and fruit essences, including apple, pear, peach, strawberry, raspberry, cherry, plum, pineapple, apricot and so forth. Flavors which have been found to be particularly useful include commercially available orange, grape, cherry and bubble gum flavors and mixtures thereof. The amount of flavoring may depend on a number of factors, including the organoleptic effect desired. Flavors may be present in an amount ranging from about 0.5% to about 3.0% by weight based upon the weight of the composition. Particularly preferred flavors are the grape and cherry flavors and citrus flavors such as orange.

The present invention is useful for capsule formulations containing low doses of Cinacalcet HCl, preferably the dose is less than 30 mg, more preferably less than 10 mg, most preferably less than 5 mg. Preferably, the powder formulation is:

| INGREDIENTS | % w/w |
|---|---|
| Cinacalcet HCl | 28.28 |
| Starch 1500 | 10.29 |
| Avicel pH 102 | 51.39 |
| Povidone K 29/32 | 3.14 |
| Polyplasdone XL | 1.89 |
| Syloid 244 FP | 5.00 |
| Total | 100.00 |

### EXAMPLES

### EXAMPLE 1: MANUFACTURE OF THE CINACALCET HCl PEDIATRIC CAPSULES

The present invention is directed to pediatric formulation of (*R*)-*N*-[-1-(1-naphthyl)-ethyl]-3-[3-(trifluoromethyl)phenyl]propan-1-amine hydrochloride (hereinafter referred to as Cinacalcet HCl) and method of administering the same.

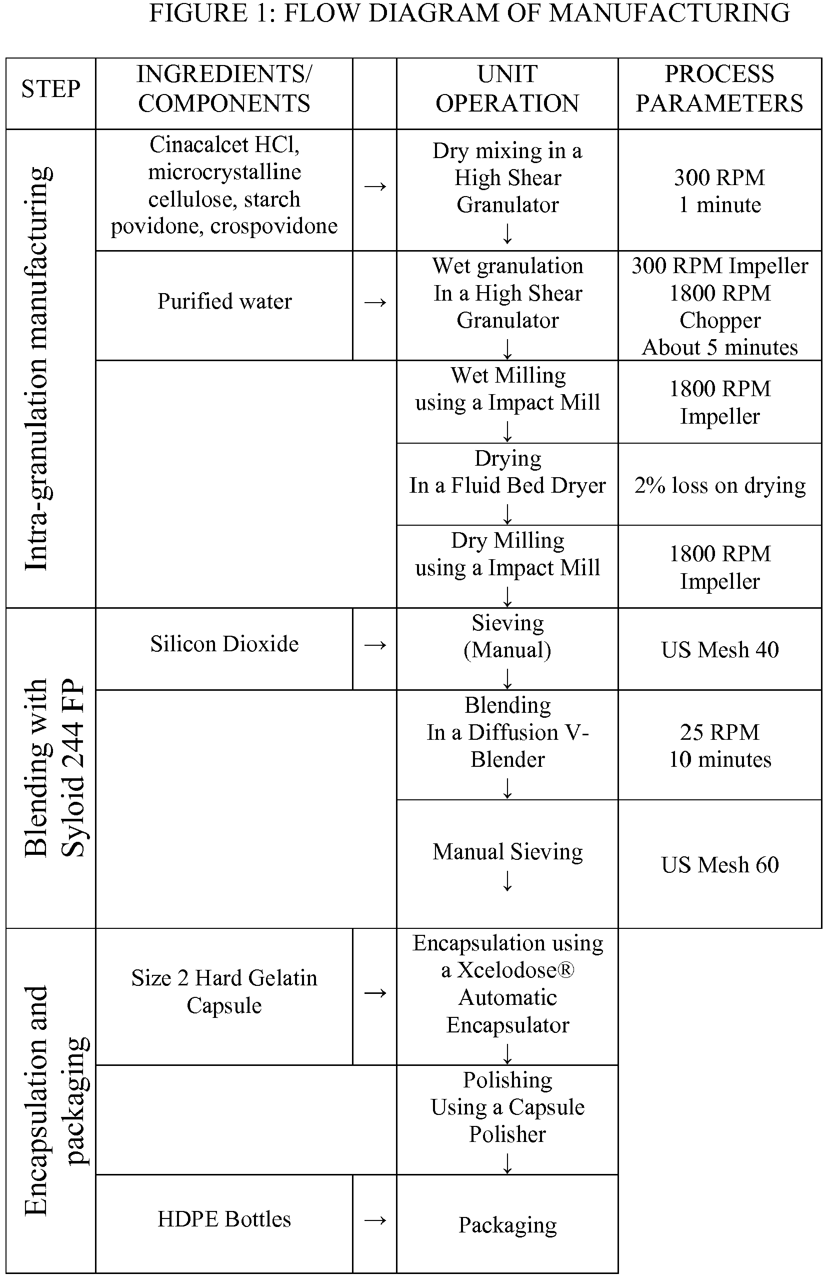

### EXAMPLE 2: FORMULATIONS AND DRUG RETENTION ASSAY STUDY

Cinacalcet HCl 1 mg pediatric capsules were prepared as in Example 1 to illustrate the present invention. The various formulations F1, F2, F3, F4, F5, and F6 shown in Table 1 were fabricated and filled into the size two hard gelatin capsules according to the techniques known in the art to achieve 1 mg dose using Automated Micro-Filling system.

**Table 1. Formulation compositions of various pediatric capsule formulations of Cinacalcet HCl**

| | Formulation composition | | | | | |
|---|---|---|---|---|---|---|
| | w/w | | | | | |
| INGREDIENTS | F1 | F2 | F3 | F4 | F5 | F6 |
| INTRA-GRANULATION | | | | | | |
| Cinacalcet HCl | 100% | 2.5% | 95.00% | 29.77% | 29.77% | 18.37% |
| Avicel PH 101 | -- | 97.5% | -- | -- | -- | -- |
| Starch 1500 | -- | -- | -- | 10.83% | 10.83% | 6.68% |
| Avicel PH 102 | -- | -- | -- | 54.1% | 54.1% | 33.38% |
| Povidone K29/32 | -- | -- | -- | 3.31% | 3.31% | 2.04% |
| Polyplasdone XL | -- | -- | -- | 1.99% | 1.99% | 1.23% |
| Syloid 244FP | -- | -- | 5.00% | -- | -- | -- |
| EXTRA-GRANULATION | -- | -- | -- | -- | -- | -- |
| Avicel PH 102 | -- | -- | -- | -- | -- | 34.30% |
| Polyplasdone XL | -- | -- | -- | -- | -- | 3.00% |
| Cab-O-Sil | -- | -- | -- | -- | -- | 0.50% |
| Magnesium Stearate | -- | -- | -- | -- | -- | 0.50% |

| | | | | | | |
|---|---|---|---|---|---|---|
| Note: Formulations F1-F4 are simple blends of formulation ingredients and F5-F6 are granular powder forms of intra-granulation and final blend of commercial Cinacalcet HCl tablet formulation | | | | | | |

Table 2 discloses the percentage of Cinacalcet HCl that was retained in capsules after sprinkling the formulations F1, F2, F3, F4, F5, and F6 shown in Table 1.

**Table 2. % Cinacalcet HCl retained in the capsules after sprinkling of various powder formulations containing Cinacalcet HCl**

| Formulations | Assay ^{a} | Recovery ^{b} | Retained ^{c} | Mass balance ^{d} |
|---|---|---|---|---|
| F1 | 99.0 | 8.83 ± 4.50 | 88.49 ± 4.59 | 97.3 ± 1.76 |
| F2 | 98.7 | 88.4 ± 2.60 | 9.8 ± 2.01 | 98.3 ± 3.72 |
| F3 | 10.1.1 | 75.59 ± 1.82 | 19.62 ± 4.92 | 95.23 ± 4.05 |
| F4 | 98.5 | 35.6 ± 6.34 | 62.9 ± 11.54 | 97.67 ± 7.54 |
| F5 | 97.0 | 48.0 ± 7.19 | 45.3 ± 10.21 | 93.3 ± 6.85 |
| F6 | 96.7 | 75.7 ± 8.58 | 21 ± 6.60 | 96.7 ± 3.63 |

| | | | | |
|---|---|---|---|---|
| ^{a.} Assay of filled capsules with a target value of 100% of claim dose ^{b.} Assay of filled powders that are sprinkled out of capsule ^{c.} Assay of capsule shells after sprinkling out of filled powders ^{d.} Sum of values of b and c | | | | |

The data presented in Table 2 indicates that the assay value of filled capsules was acceptable. However, it was found that different amounts of Cinacalcet HCl were retained in the capsules after sprinkling. Among the formulations F1, F2, F3, F4, F5, and F6 evaluated, pure drug substance-containing capsules (F1) had the least recovery, and therefore, was most retained in the capsule after sprinkling. The percentage of drug recovery increased for formulations F2 to F4 relative to F1. In addition, it was surprisingly found that the silicon dioxide was more effective and more efficient than the diluents in enhancing recovery when taking into account the relative amount of each excipient added in the formulations. Additionally, the recovery data from formulation F5 indicated that granulating Cinacalcet HCl with other excipients is more effective in enhancing drug recovery than simply mixing it with other formulation components (i.e., formulation F4).

Based on the drug retention results and the effect of silicon dioxide obtained in Table 2 above, Cinacalcet HCl formulations F7, F8, F9, F10, F12, and F13 in Table 3 were manufactured and filled into size two capsules. The formulations were sprinkled and the capsule shells were analyzed to determine how much of Cinacalcet HCl was retained after sprinkling. The results are shown in Table 4 below.

**Table 3. Formulation compositions of various pediatric capsule formulations of Cinacalcet HCl**

| | % Composition, w/w | | | | | |
|---|---|---|---|---|---|---|
| INGREDIENTS | F7 | F8 | F9 | F10 | F11 | F12 |
| Intra-granulation | | | | | | |
| Cinacalcet HCl | 2.45% | 2.38% | 29.17% | 29.17% | 28.28% | 18.00% |
| Avicel PH 101 | 94.55% | 92.6% | -- | -- | -- | -- |
| Starch 1500 | -- | -- | 10.61% | 10.61% | 10.29% | 6.55% |
| Avicel PH 102 | -- | -- | 53.02% | 53.02% | 51.39% | 32.71% |
| Povidone K29/32 | -- | -- | 3.24% | 3.24% | 3.14% | 2.00% |
| Polyplasdone XL | -- | -- | 1.95% | 1.95% | 1.89% | 1.21% |
| Syloid 244FP | 2.00% | 5.00% | 2.00% | -- | -- | -- |

| Extra-granulation | | | | | | |
|---|---|---|---|---|---|---|
| Avicel PH 102 | -- | -- | -- | -- | -- | 33.61% |
| Polyplasdone XL | -- | -- | -- | -- | -- | 2.94% |
| Cab-O-Sil | -- | -- | -- | -- | -- | 0.49% |
| Magnesium Stearate | -- | -- | -- | -- | -- | 0.49% |
| Syloid 244 FP | -- | -- | -- | 2.00% | 5.00% | 2.00% |

| | | | | | | |
|---|---|---|---|---|---|---|
| Note: Formulations F7-F9 are simple blends of formulation ingredients. Formulations F10-F11 are binary blends of intra-granulation of commercial Cinacalcet HCl tablet and syloid 244 FP, where 98% and 95% of intra-granulation were blended with 2% and 5% Syloid 244 FP, respectively. Formulation F12 is the mixture of final blend of commercial Cinacalcet HCl tablet formulation and Syloid 244 FP, where 2% Syloid 244 FP was added to the final blend of commercial tablet formulation. | | | | | | |

**Table 4: % Cinacalcet HCl retained in the capsules after sprinkling of various powder formulations containing Cinacalcet HCl**

| FORMULATIONS | ASSAY ^{a} | RECOVERY ^{b} | RETAINED ^{c} | MASS BALANCE ^{d} |
|---|---|---|---|---|
| F7 | 98.6 | 98.06 ± 1.22 | 1.75 ± 0.22 | 99.8 ± 1.41 |
| F8 | 101.8 | 98.35 ± 0.27 | 1.57 ± 0.44 | 99.97 ± 0.40 |
| F9 | 91.5 | 77.78 ± 1.97 | 15.08 ± 1.62 | 92.87 ± 0.95 |
| F10 | 93.2 | 92.25 ± 2.17 | 2.91 ± 2.44 | 95.17 ± 0.35 |
| F11 | 97.8 | 96.2 ± 1.51 | 1.6 ± 0.44 | 97.8 ± 1.67 |
| F12 | 99.8 | 96.17 ± 4.67 | 1.27 ± 0.16 | 97.43 ± 4.80 |

| | | | | |
|---|---|---|---|---|
| ^{a.} Assay of filled capsules with a target value of 100% of claim dose ^{b.} Assay of filled powders that are sprinkled out ^{c.} Assay of capsule shells after sprinkling out of filled powders ^{d.} Sum of values of b and c | | | | |

The data presented in Table 4 shows that drug recovery was improved for all formulations F7, F8, F9, F10, F12, and F13, resulting in less Cinacalcet HCl retention in the capsule shells compared to formulations F1, F2, F3, F4, F5, and F6. It should be noted that by adding 2% to 5% w/w Syloid 244 FP to formulation F2, Cinacalcet HCl retention was reduced by more than 6-fold from formulations F7 and F8; by adding 2% w/w Syloid 244 FP to formulation F4, more than 4-fold reduction of Cinacalcet HCl retention was achieved from Formulation F9. The data presented in Table 4 also indicate that addition of 2% to 5% w/w Syloid 244 FP to formulation F5 resulted in more than 18-fold reduction of Cinacalcet HCl retention from formulations F10 and F11. It can be readily appreciated by a person skilled in the art that other commercial grades of silicon dioxide, such as, but not limited to, Cab-O-Sil M-5, Cab-O-Sil S-17, Cab-O-Sil M-7D, Cab-O-Sil EH-5, Aerosil OX50, Aerosil 200VV, Aerosil 130VV, Aerosil R972V, Aerosil R974V, Wacker H2000, H2015, and H2050, can be also used in the present invention.

**Table 5: Pediatric capsule formulation containing Cinacalcet HCl**

| INGREDIENTS | % W/W |
|---|---|
| Cinacalcet HCl | 28.28 |
| Starch 1500 | 10.29 |
| Avicel pH 102 | 51.39 |
| Povidone K 29/32 | 3.14 |
| Polyplasdone XL | 1.89 |
| Syloid 244 FP | 5.00 |
| Total | 100.00 |

**Table 6. Cinacalcet HCl commercial tablet formulation**

| INGREDIENTS | % W/W |
|---|---|
| Intra-Granular Components | |
| Cinacalcet HCl | 18.37 |
| Starch 1500 | 6.68 |
| Avicel pH 102 | 33.38 |
| Povidone K 29/32 | 2.04 |
| Polyplasdone XL | 1.23 |
| SUB-TOTAL | 61.70 |

| Extra-Granular Components | |
|---|---|
| Avicel pH 102 | 34.30 |
| Polyplasdone XL | 3.00 |
| Cab-O-Sil | 0.50 |
| Magnesium Stearate | 0.50 |
| TOTAL CORE | 100.00 |

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The invention should, therefore, be determined not with reference to the above description, but should instead be determined with reference to the following appended claims, along with the full scope of equivalents to which such claims are entitled.

## Claims

1. A powder formulation comprising a therapeutically effective amount of (R)-N-[-1-(1-naphthyl)ethyl]-3-[3-(trifluoromethyl)phenyl]propan-1-amine hydrochloride admixed with at least 2% w/w to 5% w/w of silicon dioxide.

2. A capsule containing a powder formulation comprising a therapeutically effective amount of (R)-N-[-1-(1-naphthyl)ethyl]-3-[3-(trifluoromethyl)-phenyl]propan-1-amine hydrochloride admixed with at least 2% w/w to 5% w/w of silicon dioxide.

3. A powder formulation comprising (R)-N-[-1-(1-naphthyl)ethyl]-3-[3-(trifluoromethyl)-phenyl]propan-1-amine hydrochloride admixed with at least 2% w/w to 5% w/w of silicon dioxide for use in a method of treating secondary hyperparathyroidism in a pediatric patient.

4. A powder formulation consisting essentially of (R)-N-[-1-(1-naphthyl)ethyl]-3-[3-(trifluoromethyl)-phenyl]propan-1-amine hydrochloride admixed with at least 0.5% w/w to 5% w/w of silicon dioxide for use in a method of treating secondary hyperparathyroidism in a pediatric patient.

5. The powder formulation of claim 1 or 2 further comprising a binder, filler, and a disintegrant.

6. The powder formulation of any of the Claims 1, 2 and 5 wherein the amount of silicon dioxide is 5% w/w.

7. A powder formulation comprising:
| INGREDIENTS | % W/W |
|---|---|
| Cinacalcet HCl | 28.28 |
| Starch 1500 | 10.29 |
| Avicel pH 102 | 51.39 |
| Povidone K 29/32 | 3.14 |
| Polyplasdone XL | 1.89 |
| Syloid 244 FP | 5.00 |

8. The powder formulation according to claim 7, wherein the amount of cinacalcet HCl is 1 mg or is 5 mg or less.

9. The powder formulation for use according to Claim 3 or 4 wherein the powder formulation further comprises a binder, filler, and a disintegrant.

10. The powder formulation for use according to any of the Claims 3 or 4 wherein the amount of silicon dioxide in the powder formulation is 5% w/w.

11. A powder formulation as defined in claim 7 or 8:
for use in a method of treating secondary hyperparathyroidism in a pediatric patient.

12. A powder formulation consisting essentially of:
| INGREDIENTS | % W/W |
|---|---|
| Cinacalcet HCl | 28.28 |
| Starch 1500 | 10.29 |
| Avicel pH 102 | 51.39 |
| Povidone K 29/32 | 3.14 |
| Polyplasdone XL | 1.89 |
| Syloid 244 FP | 5.00 |
for use in a method of treating secondary hyperparathyroidism in a pediatric patient.

13. The powder formulation according to any of the Claims 3, 4, and 9-12 wherein the powder formulation is mixed with food or drinks prior to administering said formulation to the patient.

14. The powder formulation of any of the Claims 1, 2 and 5 wherein the amount of silicon dioxide is 5% w/w and the formulation is in granulated form.

15. A powder formulation comprising a therapeutically effective amount of (R)-N-[-1-(1-naphthyl)ethyl]-3-[3-(trifluoromethyl)phenyl]propan-1-amine hydrochloride admixed with at least 2% w/w to 5% w/w of silicon dioxide for use in the treatment of pediatric patients.

## Patentansprüche

1. Eine Pulverformulierung umfassend eine therapeutisch wirksame Menge von (R)-N-[-1-(1-naphthyl)ethyl]-3-[3-(trifluoromethyl)phenyl]propan-1-amin Hydrochlorid vermengt mit wenigstens 2 Gew.% bis 5 Gew.% Silikondioxid.

2. Eine Kapsel enthaltend eine Pulverformulierung umfassend eine therapeutisch wirksame Menge von (R)-N-[-1-(1-naphthyl)ethyl]-3-[3-(trifluoromethyl)-phenyl]propan-1-amin Hydrochlorid vermengt mit wenigstens 2 Gew.% bis 5 Gew.% Silikondioxid.

3. Eine Pulverformulierung umfassend (R)-N-[-1-(1-naphthyl)ethyl]-3-[3-(trifluoromethyl)-phenyl]propan-1-amin Hydrochlorid vermengt mit wenigstens 2 Gew.% bis 5 Gew.% Silikondioxid zur Verwendung in einem Verfahren zur Behandlung von sekundärem Hyperparathyreoidismus bei einem pädiatrischen Patienten.

4. Eine Pulverformulierung, die im Wesentlichen aus (R)-N-[-1-(1-naphthyl)ethyl]-3-[3-(trifluoromethyl)-phenyl]propan-1-amin Hydrochlorid vermengt mit wenigstens 0.5 Gew.% bis 5 Gew.% Silikondioxid besteht, zur Verwendung in einem Verfahren zur Behandlung von sekundärem Hyperparathyreoidismus bei einem pädiatrischen Patienten.

5. Die Pulverformulierung gemäß Anspruch 1 oder 2, ferner umfassend ein Bindemittel, Füllmittel und ein Sprengmittel.

6. Die Pulverformulierung gemäß einem beliebigen der Ansprüche 1, 2 und 5 wobei die Menge an Silikondioxid 5 Gew.% ist.

7. Eine Pulverformulierung umfassend:
| Inhaltsstoff | Gew.% |
|---|---|
| Cinacalcet HCl | 28.28 |
| Starch 1500 | 10.29 |
| Avicel pH 102 | 51.39 |
| Povidon K 29/32 | 3.14 |
| Polyplasdon XL | 1.89 |
| Syloid 244 FP | 5.00 |

8. Die Pulverformulierung gemäß Anspruch 7, wobei die Menge an Cinacalcet HCl 1 mg ist oder 5 mg oder weniger ist.

9. Die Pulverformulierung zur Verwendung gemäß Anspruch 3 oder 4 wobei die Pulverformulierung ferner ein Bindemittel, Füllmittel und ein Sprengmittel umfasst.

10. Die Pulverformulierung zur Verwendung gemäß Anspruch 3 oder 4 wobei die Menge an Silikondioxid in der Pulverformulierung 5 Gew.% ist.

11. Eine Pulverformulierung wie in Anspruch 7 oder 8 definiert:
zur Verwendung in einem Verfahren zur Behandlung von sekundärem Hyperparathyreoidismus bei einem pädiatrischen Patienten.

12. Eine Pulverformulierung, bestehend im Wesentlichen aus:
| Inhaltsstoff | Gew.% |
|---|---|
| Cinacalcet HCl | 28.28 |
| Starch 1500 | 10.29 |
| Avicel pH 102 | 51.39 |
| Povidon K 29/32 | 3.14 |
| Polyplasdon XL | 1.89 |
| Syloid 244 FP | 5.00 |
zur Verwendung in einem Verfahren zur Behandlung von sekundärem Hyperparathyreoidismus bei einem pädiatrischen Patienten.

13. Die Pulverformulierung gemäß einem beliebigen der Ansprüche 3, 4, und 9-12 wobei die Pulverformulierung mit Essen oder Getränken gemischt wird, bevor die genannte Formulierung dem Patienten verabreicht wird.

14. Die Pulverforrmulierung gemäß einem beliebigen der Ansprüche 1, 2 und 5 wobei die Menge an Silikondioxid 5 Gew.% ist und die Formulierung in granulierter Form vorliegt.

15. Eine Pulverformulierung umfassend eine therapeutisch wirksame Menge von (R)-N-[-1-(1-naphthyl)ethyl]-3-[3-(trifluoromethyl)phenyl]propan-1-amin Hydrochlorid vermengt mit wenigstens 2 Gew.% bis 5 Gew.% Silikondioxid zur Verwendung in der Behandlung pädiatrischer Patienten.

## Revendications

1. Formulation de poudre comprenant une quantité thérapeutiquement efficace de chlorhydrate de (R)-N-[-1-(1-naphtyl)éthyl]-3-[3-(trifluorométhyl)phényl]propan-1-amine en mélange par admixtion avec au moins 2 % en pds/pds à 5 % en pds/pds de dioxyde de silicium.

2. Capsule contenant une formulation de poudre comprenant une quantité thérapeutiquement efficace de chlorhydrate de (R)-N-[-1-(1-naphtyl)éthyl]-3-[3-(trifluorométhyl)-phényl]propan-1-amine en mélange par admixtion avec au moins 2 % en pds/pds à 5 % en pds/pds de dioxyde de silicium.

3. Formulation de poudre comprenant du chlorhydrate de (R)-N-[-1-(1-naphtyl)éthyl]-3-[3-(trifluorométhyl)-phényl]propan-1-amine en mélange par admixtion avec au moins 2 % en pds/pds à 5 % en pds/pds de dioxyde de silicium pour l'utilisation dans un procédé de traitement de l'hyperparathyroïdisme secondaire chez un patient pédiatrique.

4. Formulation de poudre constituée essentiellement de chlorhydrate de (R)-N-[-1-(1-naphtyl)éthyl]-3-[3-(trifluorométhyl)-phényl]propan-1-amine en mélange par admixtion avec au moins 0,5 % en pds/pds à 5 % en pds/pds de dioxyde de silicium pour l'utilisation dans un procédé de traitement de l'hyperparathyroïdisme secondaire chez un patient pédiatrique.

5. Formulation de poudre selon la revendication 1 ou 2 comprenant en outre un liant, une charge, et un agent délitant.

6. Formulation de poudre selon l'une quelconque des revendications 1, 2 et 5, la quantité de dioxyde de silicium étant de 5 % en pds/pds.

7. Formulation de poudre comprenant :
| INGRÉDIENTS | % en pds/pds |
|---|---|
| Cinacalcet HCl | 28,28 |
| Amidon 1500 | 10,29 |
| Avicel pH 102 | 51,39 |
| Povidone K 29/32 | 3,14 |
| Polyplasdone XL | 1,89 |
| Syloïde 244 FP | 5,00 |

8. Formulation de poudre selon la revendication 7, la quantité de cinacalcet HCl étant de 1 mg ou étant de 5 mg ou moins.

9. Formulation de poudre pour l'utilisation selon la revendication 3 ou 4, la formulation de poudre comprenant en outre un liant, une charge, et un agent délitant.

10. Formulation de poudre pour l'utilisation selon l'une quelconque des revendications 3 ou 4, la quantité de dioxyde de silicium dans la formulation de poudre étant de 5 % en pds/pds.

11. Formulation de poudre telle que définie selon la revendication 7 ou 8 :
pour l'utilisation dans un procédé de traitement de l'hyperparathyroïdisme secondaire chez un patient pédiatrique.

12. Formulation de poudre constituée essentiellement de :
| INGRÉDIENTS | % en pds/pds |
|---|---|
| Cinacalcet HCl | 28,28 |
| Amidon 1500 | 10,29 |
| Avicel pH 102 | 51,39 |
| Povidone K 29/32 | 3,14 |
| Polyplasdone XL | 1,89 |
| Syloïde 244 FP | 5,00 |
pour l'utilisation dans un procédé de traitement de l'hyperparathyroïdisme secondaire chez un patient pédiatrique.

13. Formulation de poudre selon l'une quelconque des revendications 3, 4, et 9 à 12, la formulation de poudre étant mélangée avec un aliment ou des boissons avant l'administration à ladite formulation au patient.

14. Formulation de poudre selon l'une quelconque des revendications 1, 2 et 5, la quantité de dioxyde de silicium étant de 5 % en pds/pds et la formulation se présentant sous une forme granulée.

15. Formulation de poudre comprenant une quantité thérapeutiquement efficace de chlorhydrate de (R)-N-[-1-(1-naphtyl)éthyl]-3-[3-(trifluorométhyl)phényl]propan-1-amine en mélange par admixtion avec au moins 2 % en pds/pds à 5 % en pds/pds de dioxyde de silicium pour l'utilisation dans le traitement de patients pédiatriques.
